# EUROPEAN PATENT APPLICATION

(11) **EP 3 834 785 A1**
(43) Date of publication of application: **16.06.2021**
(21) Application number: 19848571.6
(22) Date of filing: 01.08.2019
(51) Int. Cl.: A61F 5/56, A61F 5/058

(54) **BRACE FOR SUPPORTING JAW ANGLE, AND JAW ANGLE CORRECTING APPARATUS INCLUDING SAME**

(30) Priority: 06.08.2018 KR 20180091357
(71) Applicant: Choi, Jong-Soo, Gangnam-gu, Seoul 06330 (KR)
(72) Inventor: Choi, Jong-Soo, Gangnam-gu, Seoul 06330 (KR)
(74) Representative: Viering, Jentschura & Partner mbB Patent- und Rechtsanwälte
(86) International application number: PCT/KR2019/009617
(87) International publication number: WO 2020/032479

(57) **Abstract**

A jaw support brace includes a mounting portion extending from a middle region of a user's chest along a pair of collarbones and being supported on the collarbones, at least one elastic supporting portion extending upwardly from the mounting portion along at least one side surface of a neck, and a jaw resting portion extending below a mandible from the elastic supporting portion and supporting a jaw at a desired angle by an elastic supporting force of the elastic supporting portion

## Description

### TECHNICAL FIELD

The present invention relates to a jaw support brace. More particularly, the present invention relates to a jaw angle correcting device capable of help support a user's jaw angle.

### BACKGROUND ART

Generally, snoring and sleep apnea simply deteriorate the quality of sleep, and not only manifest various systemic diseases such as interpersonal disorders, narcolepsy, heart disease, respiratory disease, etc., but also seriously affect human life, such as attention deficit in adolescence. In particular, in the case of obstructive sleep apnea, severe snoring is accompanied.

In order to relieve such snoring, intra-oral, that is, a brace disposed in the mouth, or extra-oral, that is, a brace placed outside the mouth may be used. However, existing braces are inconvenient to use and have limitations in spatial use.

### DISCLOSURE OF THE INVENTION

### PROBLEMES TO BE SOLVED

An object of the present invention provides a jaw support brace that supports a jaw angle at a desired angle and is provided in a structure suitable for individual physical structure.

Another object of the present invention provides a correction device for jaw angle support including the jaw support brace.

### MEANS TO SOLVE THE PROBLEMS

According to example embodiments, a jaw support brace includes a mounting portion extending from a middle region of a user's chest along a pair of collarbones and being supported on the collarbones, at least one elastic supporting portion extending upwardly from the mounting portion along at least one side surface of a neck, and a jaw resting portion extending below a mandible from the elastic supporting portion and supporting a jaw at a desired angle by an elastic supporting force of the elastic supporting portion.

In example embodiments, the jaw support brace may include a wire extending from a front chest through the side surface of the neck to a lower portion of the mandible to form one closed loop.

In example embodiments, the wire may include a material that is bendable to follow a natural curvature of the user's individual neck length and jaw angle.

In example embodiments, the jaw support brace may further include a tube that surrounds the wire and includes a material having skin affinity

In example embodiments, the tube may include silicon.

In example embodiments, the elastic supporting portion may include first and second elastic supporting portions extending upwardly from both end portions of the mounting portion along left and right side surfaces of the neck, and the jaw support brace may further include first and second auxiliary supporting portions respectively extending in a U-shape from the first and second elastic supporting portions to the mandibles under ears through depressions behind the ears to be connected to both end portions of the jaw resting portion.

In this case, both end portions of each of the first and second auxiliary supporting portions may extend parallel with each other and may be spaced apart by a predetermined distance.

In example embodiments, an angle between the mounting portion and the first and second elastic supporting portions extending therefrom may be greater than at least 90 degrees.

In example embodiments, an angle between the first elastic supporting portion connected to an end portion of the first auxiliary supporting portion and the jaw resting portion connected to another portion of the first auxiliary supporting portion and an angle between the second elastic supporting portion connected to an end portion of the second auxiliary supporting portion and the jaw resting portion connected to another portion of the second auxiliary supporting portion may be greater than at least 90 degrees.

In example embodiments, the elastic supporting portion may extend upwardly from a middle portion of the mounting portion along a front of the neck to be connected to middle portion of the jaw resting portion.

In example embodiments, an angle between the mounting portion and the elastic supporting portion extending therefrom may be less than 90 degrees. An angle between the elastic supporting portion and the jaw resting portion may be less than 90 degrees.

According to example embodiments, a correction device for jaw angle support includes a jaw support brace configured to be worn between a user's collarbone and a jaw to support the jaw at a desired angle, and a neck protector configured to be worn around the jaw support brace to surround a circumference of a neck. The jaw support brace includes a mounting portion extending from a middle region of a chest along a pair of the collarbones and being supported on the collarbones, at least one elastic supporting portion extending upwardly from the mounting portion along at least one side surface of the neck, and a jaw resting portion extending below a mandible from the elastic supporting portion and supporting the jaw by an elastic supporting force of the elastic supporting portion.

In example embodiments, the neck protector may include a first covering portion covering a portion of the neck, a second covering portion covering the rest of the neck, and a coupling member that couples the first and second covering portions to each other to be worn around the neck.

### EFFECTS OF THE INVENTION

A jaw support brace according to example embodiments may include a mounting portion extending from a middle region of a chest along a pair of collarbones, at least one elastic supporting portion extending upwardly from the mounting portion along at least one side surface of a neck, and a jaw resting portion extending below a mandible from the elastic supporting portion and supporting a jaw by an elastic supporting force of the elastic supporting portion. The jaw support brace may include a wire extending from a front chest through a side surface of the neck to a lower portion of the mandible to form one closed loop.

The wire may be bent and adjusted to fit the user's individual body structure to form the components of the jaw support brace. An angle of the user's jaw may be adjusted to a desired angle, and because uncomfortable pressure does not be applied to the chin, neck and rear portions of ears, the jaw support brace may be can be worn for a long time.

The jaw support brace capable of maintaining the jaw at the desired angle may be used as a disk patient brace for a neck disk patient.

However, the effect of the invention may not be limited thereto, and may be expanded without being deviated from the concept and the scope of the present invention.

### BRIEF DESCRIPTION OF THE DRAWINGS

FIG. 1 is a perspective view illustrating a jaw support brace in accordance with example embodiments.
FIG. 2 is a plan view illustrating the jaw support brace in FIG. 1.
FIG. 3 is a rear view illustrating the jaw support brace in FIG. 1.
FIG. 4 is a side view illustrating the jaw support brace FIG. 1.
FIG. 5 is a side view illustrating a jaw supporting forward traction force generated by the jaw support brace in FIG. 1.
FIG. 6 is a cross-sectional view illustrating a portion of the jaw support brace in FIG. 1.
FIG. 7 is a perspective view illustrating a state in which the jaw support brace of FIG. 1 is used.
FIG. 8 is a side view of FIG. 7.
FIG. 9 is a perspective view illustrating a jaw support brace in accordance with example embodiments.
FIG. 10 is a plan view illustrating the jaw support brace in FIG. 9.
FIG. 11 is a rear view illustrating the jaw support brace in FIG. 9.
FIG. 12 is a side view illustrating the jaw support brace FIG. 9.
FIG. 13 is a perspective view illustrating a configuration of the jaw support brace in FIG. 9.
FIG. 14 is an exploded perspective view illustrating a neck protector of a correction device for jaw angle support in accordance with example embodiments.
FIG. 15 is a plan view illustrating the neck protector in FIG. 14.
FIG. 16 is a side view illustrating a state in which the correction apparatus for jaw angle support including the neck protector of FIG. 14 is used.

### BEST MODE FOR CARRYING OUT THE INVENTION

Hereinafter, preferable embodiments of the present invention will be explained in detail with reference to the accompanying drawings.

In the drawings, the sizes and relative sizes of components or elements may be exaggerated for clarity.

It will be understood that, although the terms first, second, third, etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, a first element, component, region, layer or section discussed below could be termed a second element, component, region, layer or section without departing from the teachings of example embodiments.

The terminology used herein is for the purpose of describing particular example embodiments only and is not intended to be limiting of example embodiments. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which example embodiments belong. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Example embodiments may, however, be embodied in many different forms and should not be construed as limited to example embodiments set forth herein. Rather, these example embodiments are provided so that this disclosure will be thorough and complete, and will fully convey the scope of example embodiments to those skilled in the art.

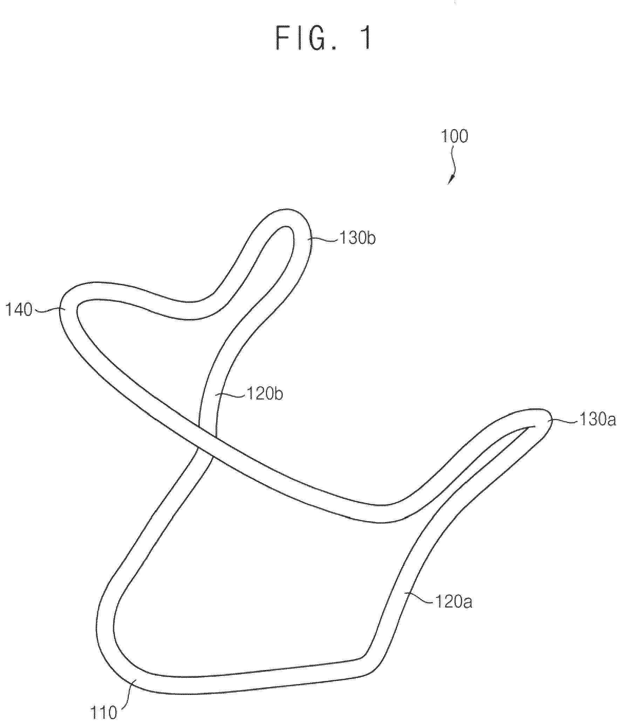

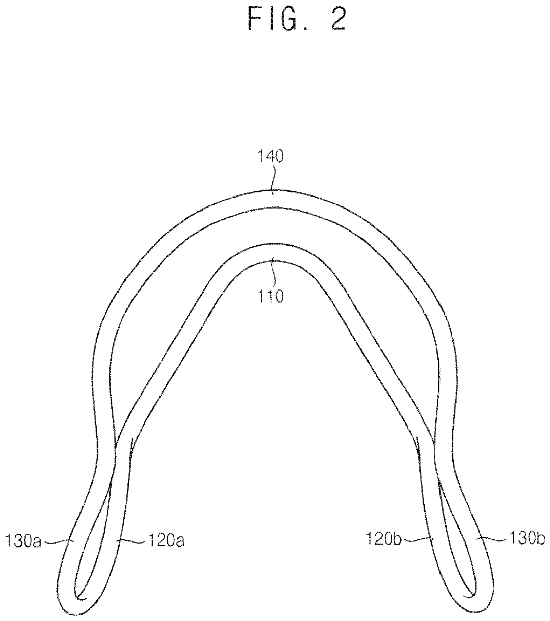

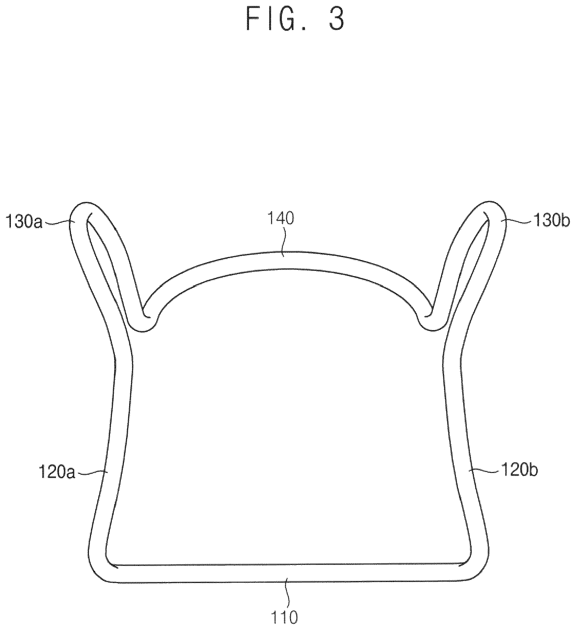

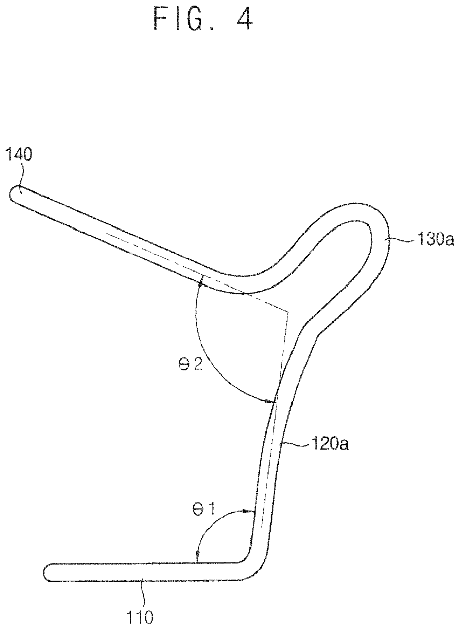

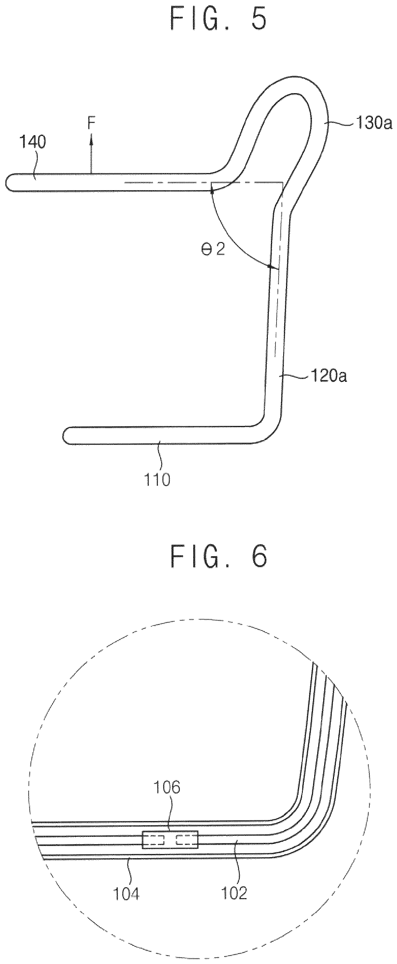

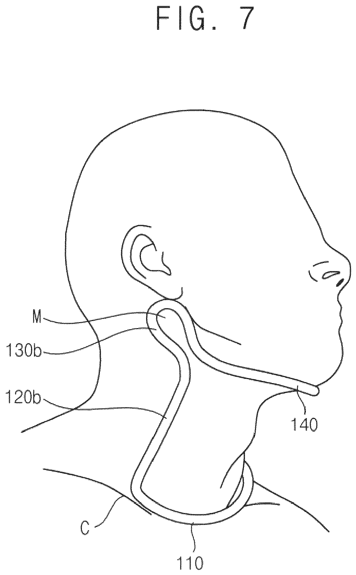

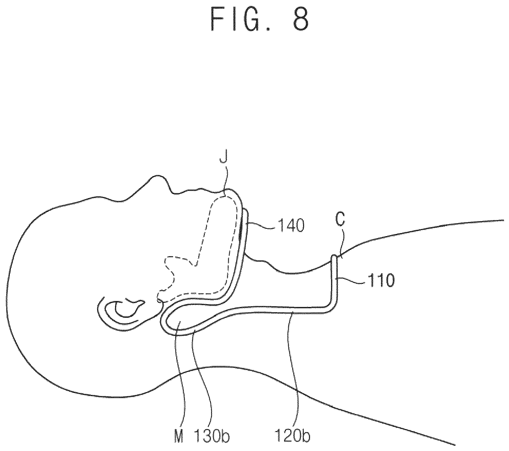

FIG. 1 is a perspective view illustrating a jaw support brace in accordance with example embodiments. FIG. 2 is a plan view illustrating the jaw support brace in FIG. 1. FIG. 3 is a rear view illustrating the jaw support brace in FIG. 1. FIG. 4 is a side view illustrating the jaw support brace FIG. 1. FIG. 5 is a side view illustrating a jaw supporting forward traction force generated by the jaw support brace in FIG. 1. FIG. 6 is a cross-sectional view illustrating a portion of the jaw support brace in FIG. 1. FIG. 7 is a perspective view illustrating a state in which the jaw support brace of FIG. 1 is used. FIG. 8 is a side view of FIG. 7.

Referring to FIGS. 1 to 8, a jaw support brace 100 may include a mounting portion 110, at least one elastic supporting portion 120a, 120b, and a jaw resting portion 140. Additionally, the jaw support brace 100 may further include at least one auxiliary supporting portion 130a, 130b.

In example embodiments, the jaw support brace 100 may be used as a sleep aid for preventing snoring and sleep apnea syndrome. The jaw support brace 100 may be used as a brace for a disk patient such as a neck disk patient.

In particular, the mounting portion 110 may be configured to extend from a middle region of a user's chest along a pair of collarbones C and be supported on the collarbones. When viewed in the plan view, the mounting portion 110 may have a shape extending in a V-shape. Accordingly, both end portions of the mounting portion 110 may be seated respectively in recessed portions between a neck and the chest and adjacent to a pair of the collarbones.

In example embodiments, the jaw support brace 100 may include two first and second elastic supporting portions 120a, 120b. The first and second elastic supporting portions 120a, 120b may extend upwardly from both end portions of the mounting portion 110 along left and right side surfaces of the neck.

As illustrated in FIG. 5, when viewed in the side view, the first and second elastic supporting portions 120a, 120b may extend upwardly toward a back of an ear to form an angle θ1 between the mounting portion 110 and the first and second elastic supporting portions 120a, 120b to be greater than at least 90 degrees. For example, the angle θ1 between the mounting portion 110 and the first and second elastic supporting portions 120a, 120b may be within an angle range of 90 degrees to 120 degrees.

In addition, as illustrated in FIG. 2, when viewed in the rear view, the first and second elastic supporting portions 120a, 120b may extend toward the left and right sides of the neck, that is, obliquely toward the inside.

In example embodiments, the jaw support brace 100 may further include first and second auxiliary supporting portions 130a, 130b between the first and second elastic supporting portions 120a, 120b and the jaw resting portion 140. The first and second auxiliary supporting portions 130a, 130b may extend in a U-shape from the first and second elastic supporting portions 120a, 120b respectively to a mandible J under the ear through a depression behind the ear.

As illustrated in FIG. 5, the first and second auxiliary supporting portions 130a, 130b may extend upwardly from the first and second elastic supporting portions 120a, 120b toward the rear, go by the depression behind the ear and extend downwardly toward the front to form the U-shape. Both end portions of the first auxiliary supporting portion 130a may extend parallel with each other and may be spaced apart by a predetermined distance. Both end portions of the second auxiliary supporting portion 130b may extend parallel with each other and may be spaced apart by a predetermined distance. When the U-shaped first and second auxiliary supporting portions 130a, 130b are pressed in a direction closer to each other, each of first and second auxiliary supporting portions may have an elastic force to return to its original position.

In addition, the U-shaped first and second auxiliary supporting portions 130a, 130b may be formed to be located near a mastoid process M behind the ear. Both end portions of each of the first and second auxiliary supporting portions 130a, 130b may be arranged to be spaced apart from each other, thereby maintaining a smooth flow of blood through a blood vessel (acupoint in the anterior region of the neck, in the depression posteroinferior to the mastoid process) and relieving stress so that it may not be uncomfortable even if a user wear it for a long time.

The jaw resting portion 140 may extend below the mandible from the first and second auxiliary supporting portions 130a and 130b and support the jaw at a desired angle by the elastic supporting force of the first and second elastic supporting portions 120a, 120b and the first and second auxiliary supporting portions 130a, 130b. The jaw resting portion 140 may have a shape extending in a U-shape when viewed in the plan view. The jaw resting portion 140 may extend laterally from a lower portion of the mandible. Accordingly, the jaw resting portion 140 may apply a forward traction force to a portion of the jaw below the mandible to support the jaw at a desired angle without sagging down the mandible.

As illustrated in FIG. 5, when viewed in the side view, an angle Θ2 between the first elastic supporting portion 130a connected to an end portion of the first auxiliary supporting portion 130a and the jaw resting portion 140 connected to another portion of the first auxiliary supporting portion 130a may be greater than at least 90 degrees. For example, the angle θ2 between the first elastic supporting portion 130a and the jaw resting portion 140 may be within an angle range of 100 degrees to 130 degrees.

Similarly, an angle between the second elastic supporting portion 130b connected to an end portion of the second auxiliary supporting portion 130b and the jaw resting portion 140 connected to another portion of the second auxiliary supporting portion 130b may be greater than at least 90 degrees. For example, the angle between the second elastic supporting portion 130b and the jaw resting portion 140 may be within an angle range of 100 degrees to 130 degrees.

As illustrated in FIG. 6, as the mandible sags or the jaw goes down, the jaw resting portion 140 may go down, and accordingly, the U-shaped first and second auxiliary supporting portions 130a, 130b may be pressed in a direction closer to each other. In this case, since the first and second elastic supporting portions 120a, 120b and the first and second auxiliary supporting portions 130a, 130b have an elastic force to return to their original positions, the jaw resting portion 140 may apply a forward traction force F on the jaw to support the jaw at a desired angle without sagging down the mandible.

In example embodiments, the jaw support brace 100 may include a wire 102 extending from the front chest through the left and right sides of the neck to the lower portion of the mandible to form one closed loop. The wire 102 may be bent along the chest, the neck and the jaw to form the mounting portion 110, the first and second elastic supporting portions 120a, 120b, the first and second auxiliary supporting portions 130a, 130b and the jaw resting portion 140. The wire 102 may include a material that is bendable to follow a natural curvature of the user's individual neck length and jaw angle. A thickness and strength of the wire 102 may be determined in consideration of a required forward traction force, a user's individual body structure, etc.

As illustrated in FIG. 7, the wire 102 may be cut to have a desired length, and both ends of the wire 102 may be fixed by a fixing clip (or fixing band) 106 to form a closed loop. Accordingly, the wire 102 may be bent and adjusted to fit the user's individual body structure, and then, an unnecessary portion may be cut.

In addition, the jaw support brace 100 may further include a tube 104 that surrounds the wire 102 and includes a material having skin affinity. For example, the tube 104 may include silicon, plastic, or the like having skin affinity. Alternatively, the jaw support brace 100 may include a sponge-like material surrounding the wire 102 instead of the tube.

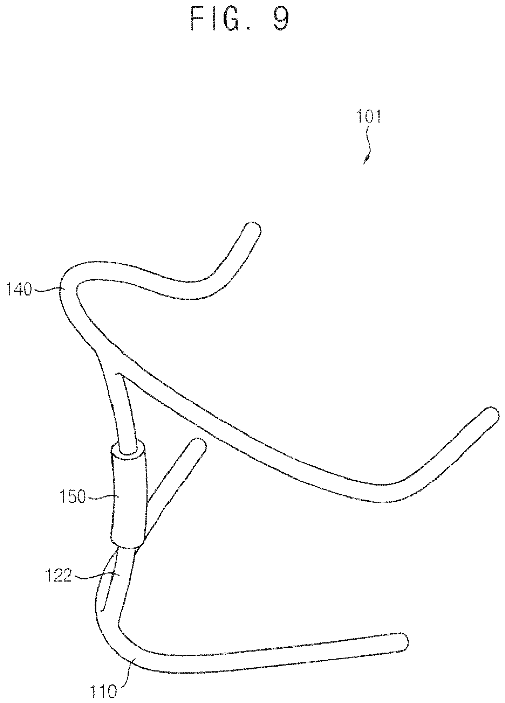

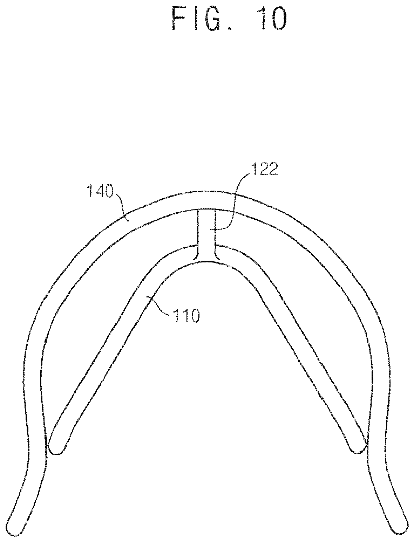

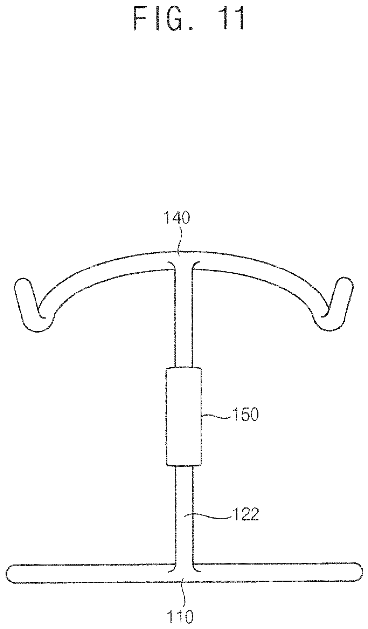

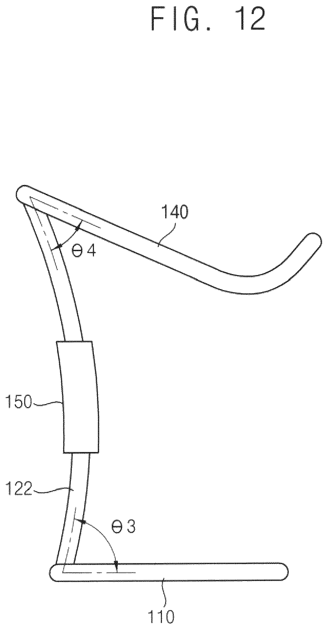

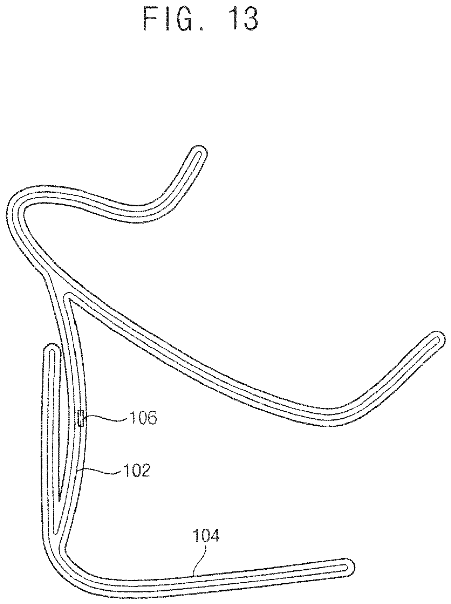

FIG. 9 is a perspective view illustrating a jaw support brace in accordance with example embodiments. FIG. 10 is a plan view illustrating the jaw support brace in FIG. 9. FIG. 11 is a rear view illustrating the jaw support brace in FIG. 9. FIG. 12 is a side view illustrating the jaw support brace FIG. 9. FIG. 13 is a perspective view illustrating a configuration of the jaw support brace in FIG. 9.

Referring to FIGS. 9 to 13, a jaw support brace 101 may include a mounting portion 110, an elastic supporting portion 122 and a jaw resting portion 140.

In particular, the mounting portion 110 may be configured to extend from a middle region of a user's chest along a pair of collarbones C and be supported on the collarbones. When viewed in the plan view, the mounting portion 110 may have a shape extending in a V-shape.

The elastic supporting portion 122 may extend upwardly from a middle portion of the mounting portion 110 along a front of a neck. An end portion of the elastic supporting portion 122 may be connected to the middle portion of the mounting portion 110, and another portion of the elastic supporting portion 122 may be connected to a middle portion of the jaw resting portion 140.

As illustrated in FIG. 12, when viewed in the side view, the elastic supporting portion 122 may extend upwardly toward a jaw and be spaced apart from the front of the neck to form an angle θ3 between the mounting portion 110 and the elastic supporting portion 122 to be less than 90 degrees. Additionally, the elastic supporting portion 122 may be curved toward the front of the neck, that is, toward the inside, to have an overall concave shape.

The jaw resting portion 140 may extend toward both sides of the neck below the mandible and support the jaw at a desired angle by an elastic supporting force of the elastic supporting portion 122. The jaw resting portion 140 may have a shape extending in a U-shape, when viewed in the plan view. The jaw resting portion 140 may extend laterally from a lower portion of the mandible. Accordingly, the jaw resting portion 140 may apply a forward traction force to a lower portion of the jaw below the mandible to support the jaw at a desired angle without sagging down the mandible.

As illustrated in FIG. 12, when viewed in the side view, an angle θ4 between the elastic supporting portion 122 and the jaw resting portion 140 may be less than 90 degrees. For example, the angle θ4 between the elastic supporting portion 122 and the jaw resting portion 140 may be within an angle range of 50 degrees to 80 degrees.

As the mandible sags or the jaw goes down, the jaw resting portion 140 may go down. At this time, since the elastic supporting portion 122 has an elastic force to return to its original position, the jaw resting portion 140 may apply a forward traction force F on the jaw to support the jaw at a desired angle without sagging down the mandible.

As illustrated in FIG. 13, the jaw support brace 100 may include a wire 102 extending from the front chest through the front side of the neck to the lower portion of the mandible to form one closed loop. The wire 102 may be bent along the chest, the neck and the jaw to form the mounting portion 110, the elastic supporting portion 122 and the jaw resting portion 140. The wire 102 may be cut to have a desired length, and both ends of the wire 102 may be fixed by a fixing clip (or fixing band) 106 to form a closed loop. Accordingly, the wire 102 may be bent and adjusted to fit the user's individual body structure, and then, an unnecessary portion may be cut.

In addition, the jaw support brace 101 may further include a tube 104 that surrounds the wire 102 and includes a material having skin affinity. For example, the tube 104 may include silicon, plastic, or the like having skin affinity. Alternatively, the jaw support brace 100 may include a sponge-like material surrounding the wire 102 instead of the tube.

The jaw support brace 101 may further include a handle portion 150 surrounding a middle portion of the elastic supporting portion 122. The user may grip the handle portion 150 and place the jaw support brace 101 at a desired position between the front chest and the chin. The handle portion 150 may also serve as a reinforcing member that enhances the elastic force of the elastic supporting portion 122.

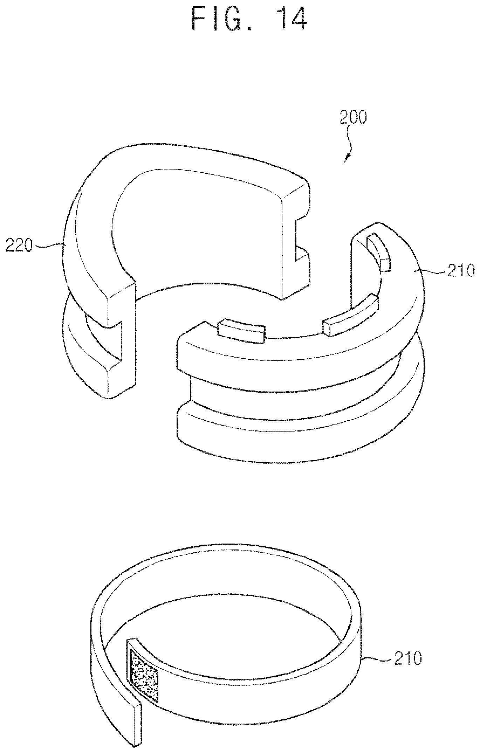

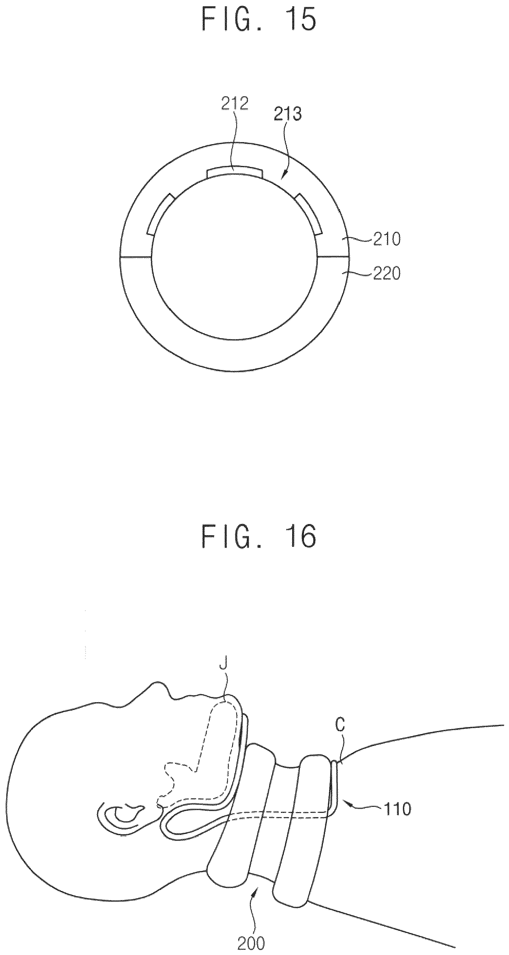

FIG. 14 is an exploded perspective view illustrating a neck protector of a correction device for jaw angle support in accordance with example embodiments. FIG. 15 is a plan view illustrating the neck protector in FIG. 14. FIG. 16 is a side view illustrating a state in which the correction apparatus for jaw angle support including the neck protector of FIG. 14 is used. The correction device for jaw angle support may further include a neck protector used together with the jaw support brace described with reference to FIGS. 1 to 8 or FIGS. 9 to 13.

Referring to FIGS. 14 to 16, a correction device for jaw angle support may include a jaw support brace 100, 101 and a neck protector 200. The neck protector 200 may be worn around the jaw support brace 100, 101 to surround a circumference of a neck.

In particular, the neck protector 200 may include a cover portion provided to surround the neck. The cover portion may include a first covering portion 210 covering a portion of the neck and a second covering portion 220 covering the rest of the neck. Accordingly, the first and second covering portions 210, 220 may be coupled to each other so as to surround the entire circumference of the neck. The neck protector 200 may further include a coupling member 230 that couples the first and second covering portions 210, 220 to each other to be worn around the neck.

The first covering portion 210 of the cover portion may be a semi arc-shaped front cover covering a front circumference of the neck, and the second covering portion 220 of the cover portion may be a semi arc-shaped rear cover covering a rear circumference of the neck. The cover portion may be formed of a soft synthetic resin material that can be deformed by an external force when worn. For example, the cover portion may include a sponge-like material.

The coupling member 230 may include a fixing band accommodated in grooves formed on outer surfaces of the first and second covering portion 210, 220. The fixing band may be formed of an elastic material. The first and second covering portions 210, 220 may be detachable from each other by the fixing band. A hook and a hook made of adhesive clothes such as Velcro tape at both end portions of the fixing band may be paired and attached to each other. Alternatively, the coupling member may include male and female fastening clips to couple end portions of the first and second covering portions 210, 220 adjacent to each other. However, the coupling member may not be limited thereto, and it will be understood that members having various structures for wearing the cover portion around the neck may be used.

As illustrated in FIG. 16, the cover portion of the neck protector 200 may be provided to surround at least a portion of the jaw support brace 100, for example, first and second elastic supporting portions 120a, 120b. The jaw support brace 100 (e.g., the first and second elastic supporting portions) may be integrally formed on an inner side of the neck protector 200. Accordingly, the jaw support brace 100 may be fixed to an inner surface of the neck protector 200.

The neck protector 200 may be worn together with the jaw support brace 100, 101 such that the neck protector 200 is worn around the circumference of the jaw support brace 100, 101 to surround the neck. Accordingly, it may be possible to securely fix the jaw support brace 100, 101 and protect a patient's cervical spine.

As illustrated in FIGS. 14 and 15, at least one protruding protrusion 212 may be formed on an upper surface or a lower surface of the first cover portion. A seating groove 213 may be defined by the protruding protrusion 212, and a portion of the jaw support brace 100, for example, a jaw resting portion 140, may be disposed to extend along the seating groove 213. Accordingly, the jaw support brace 100, 101 may be supported more firmly within the neck protector 200.

The present invention has been explained with reference to preferable embodiments, however, those skilled in the art may understand that the present invention may be modified or changed without being deviated from the concept and the scope of the present invention disclosed in the following claims.

### <The description of the reference numerals>

100, 101: jaw support brace 102: wire
104: tube 106: fixing clip
110: mounting portion 120a, 120b, 122: elastic supporting portion
130a, 130b: auxiliary supporting portion 140: jaw resting portion
200: neck protector 210: first covering portion
220: second covering portion 230: coupling member

## Claims

1. A jaw support brace, comprising:
a mounting portion extending from a middle region of a user's chest along a pair of collarbones and being supported on the collarbones;
at least one elastic supporting portion extending upwardly from the mounting portion along at least one side surface of a neck; and
a jaw resting portion extending below a mandible from the elastic supporting portion and supporting a jaw at a desired angle by an elastic supporting force of the elastic supporting portion.

2. The jaw support brace of claim 1, wherein the jaw support brace comprises a wire extending from a front chest through the side surface of the neck to a lower portion of the mandible to form one closed loop.

3. The jaw support brace of claim 2, wherein the wire includes a material that is bendable to follow a natural curvature of the user's individual neck length and jaw angle.

4. The jaw support brace of claim 3, further comprising:
a tube that surrounds the wire and includes a material having skin affinity.

5. The jaw support brace of claim 4, wherein the tube includes silicon.

6. The jaw support brace of claim 1, wherein the elastic supporting portion comprises first and second elastic supporting portions extending upwardly from both end portions of the mounting portion along left and right side surfaces of the neck, and
the jaw support brace further comprising:
first and second auxiliary supporting portions respectively extending in a U-shape from the first and second elastic supporting portions to the mandibles under ears through depressions behind the ears to be connected to both end portions of the jaw resting portion.

7. The jaw support brace of claim 6, wherein both end portions of each of the first and second auxiliary supporting portions extend parallel with each other and are spaced apart by a predetermined distance.

8. The jaw support brace of claim 6, wherein an angle between the mounting portion and the first and second elastic supporting portions extending therefrom is greater than at least 90 degrees.

9. The jaw support brace of claim 6, wherein an angle between the first elastic supporting portion connected to an end portion of the first auxiliary supporting portion and the jaw resting portion connected to another portion of the first auxiliary supporting portion and an angle between the second elastic supporting portion connected to an end portion of the second auxiliary supporting portion and the jaw resting portion connected to another portion of the second auxiliary supporting portion are greater than at least 90 degrees.

10. The jaw support brace of claim 1, wherein the elastic supporting portion extends upwardly from a middle portion of the mounting portion along a front of the neck to be connected to middle portion of the jaw resting portion.

11. The jaw support brace of claim 10, wherein an angle between the mounting portion and the elastic supporting portion extending therefrom is less than 90 degrees.

12. The jaw support brace of claim 10, wherein an angle between the elastic supporting portion and the jaw resting portion is less than 90 degrees.

13. A correction device for jaw angle support, the correction device comprising:
a jaw support brace configured to be worn between a user's collarbone and a jaw to support the jaw at a desired angle; and
a neck protector configured to be worn around the jaw support brace to surround a circumference of a neck,
wherein the jaw support brace comprises:
a mounting portion extending from a middle region of a chest along a pair of the collarbones and being supported on the collarbones;
at least one elastic supporting portion extending upwardly from the mounting portion along at least one side surface of the neck; and
a jaw resting portion extending below a mandible from the elastic supporting portion and supporting the jaw by an elastic supporting force of the elastic supporting portion.

14. The correction device for jaw angle support of claim 13, wherein the neck protector comprises a first covering portion covering a portion of the neck, a second covering portion covering the rest of the neck, and a coupling member that couples the first and second covering portions to each other to be worn around the neck.
